# EUROPEAN PATENT SPECIFICATION

(11) **EP 4 178 606 B1**
(45) Date of publication and mention of the grant of the patent: **17.09.2025**
(21) Application number: 21838650.6
(22) Date of filing: 29.06.2021
(51) Int. Cl.: A61K 38/26, A61K 9/70, A61K 38/28, A61P 3/10

(54) **THERAPEUTIC HYBRID MICRONEEDLE PATCH FOR THE DELIVERY OF INSULIN AND GLUCAGON**
THERAPEUTISCHES HYBRIDES MIKRONADELPFLASTER ZUR VERABREICHUNG VON INSULIN UND GLUCAGON
TIMBRE THÉRAPEUTIQUE À MICRO-AIGUILLES HYBRIDES D'ADMINISTRATION D'INSULINE ET DE GLUCAGON

(30) Priority: 07.07.2020 US 202063048913 P
(43) Date of publication of application: 17.05.2023
(73) Proprietor: The Regents of University of California, Oakland, CA 94607-5200 (US)
(72) Inventor: GU, Zhen, Los Angeles, CA 90095-7191 (US); WANG, Zejun, Los Angeles, CA 90095-7191 (US)
(74) Representative: Ipsilon Benelux
(86) International application number: PCT/US2021/039693
(87) International publication number: WO 2022/010698

(56) References cited:
- WO-A1-2018/085809
- WO-A1-2018/165294
- WO-A1-2020/041787
- WO-A1-2020/093173
- US-A1- 2019 000 966
- US-A1- 2020 330 562
- PEDERSEN CHRISTINA ET AL: "Dual treatment with a fixed ratio of glucagon and insulin increases the therapeutic window of insulin in diabetic rats", PHYSIOLOGICAL REPORTS, vol. 6, no. 6, 1 March 2018 (2018-03-01), US, pages e13657, XP093102067, ISSN: 2051-817X, Retrieved from the Internet <URL:https://www.ncbi.nlm.nih.gov/pmc/articles/PMC5875541/pdf/PHY2-6-e13657.pdf> DOI: 10.14814/phy2.13657
- WANG ZEJUN, WANG JINQIANG, LI HONGJUN, YU JICHENG, CHEN GUOJUN, KAHKOSKA ANNA R., WU VALERIE, ZENG YI, WEN DI, MIEDEMA JAYSON R., : "Dual self-regulated delivery of insulin and glucagon by a hybrid patch", PROCEEDINGS OF THE NATIONAL ACADEMY OF SCIENCES, NATIONAL ACADEMY OF SCIENCES, vol. 117, no. 47, 24 November 2020 (2020-11-24), pages 29512 - 29517, XP055898212, ISSN: 0027-8424, DOI: 10.1073/pnas.2011099117

## Description

### Technical Field

The technical field generally relates to a therapeutic hybrid microneedle patch or patch system that incorporates microneedles for the delivery of insulin and glucagon to a mammalian subject.

### Background

WO 2020/041787 A1 discloses a therapeutic microneedle patch with insulin or glucagon loaded copolymers.

PEDERSEN CHRISTINA ET AL, "Dual treatment with a fixed ratio of glucagon and insulin increases the therapeutic window of insulin in diabetic rats", PHYSIOLOGICAL REPORTS, US, vol. 6, no. 6, doi:10.14814/phy2.13657, ISSN 2051-817X, (20180301), page e13657, URL: https://www.ncbi.nlm.nih.gov/pmc/articles/PMC5875541/pdf/PHY2-6-e13657.pdf, discloses a dual treatment in diabetic rats by administering a mixture of insulin and glucagon with a fixed ratio thereof for reducing the risk of hypoglycemia.

Pancreatic islets play a critical role in blood glucose homeostasis through the reciprocal regulation of insulin produced from *β*-cells and glucagon secreted from *α*-cells. Type 1 diabetes (T1D) is an autoimmune disease in which the pancreatic *β*-cells are destroyed and there is a deficiency in insulin secretion. Type 2 diabetes (T2D), on the other hand, is a metabolic disorder resulting from insulin resistance and *β*-cell dysfunction with impaired insulin secretion. Current treatment methods for both T1D and advanced T2D address insulin deficiency and include modalities such as subcutaneous insulin injection or infusion, endogenous insulin stimulation, and novel glucose-responsive insulin administrations. Although these treatments can be effective in treating hyperglycemia, they carry the risk of hypoglycemia and therefore require patients to monitor and rapidly respond to episodes of low blood sugar to prevent progression to seizure, coma, or death.

It has recently become recognized that the destruction of *β*-cells may disrupt other islet cell types and lead to the hyper- or hyposecretion of glucagon from the *α*-cells. *α*-cell dysfunction can further exacerbate hyperglycemia among individuals with diabetes and importantly, may increase the risk for severe hypoglycemia due to an abnormal counterregulatory response during insulin treatment. Therefore, researchers have focused on exploring approaches to reprogram and modulate *α*-cell function. Unfortunately, compared with the advances of glucose-responsive insulin delivery systems to address insulin deficiency due to *β*-cell destruction or dysfunction, the development of therapeutic systems to treat *α*-cell dysfunction and mitigate the associated risk for acute hypoglycemia remains challenging.

### Summary

The invention is directed to a therapeutic hybrid microneedle patch for delivering insulin and glucagon to living mammalian tissue, according to claim 1, including a base having a plurality of microneedles extending away from the surface of the base, wherein a first plurality of microneedles comprise a biocompatible polymer loaded with insulin and a second plurality of microneedles comprise the biocompatible polymer loaded with glucagon. In one embodiment, the first plurality of microneedles may be located at a first region of the therapeutic hybrid microneedle patch while the second plurality of microneedles may be located at a second region of the therapeutic hybrid microneedle patch. In other embodiments, the different microneedle types (e.g., insulin-containing or glucagon-containing) may be interspersed with one another. Further developments of the invention are according to dependent claims 2-6.

The invention is also directed to a therapeutic hybrid microneedle patch system for delivering insulin and glucagon to living mammalian tissue, according to claim 7, including a first patch comprising a base having a plurality of microneedles extending away from the surface of the base, wherein the microneedles of the first patch comprise a biocompatible polymer loaded with insulin and a second patch comprising a base having a plurality of microneedles extending away from the surface of the base, wherein the microneedles of the second patch comprise a biocompatible polymer loaded with glucagon. The first and second patches are applied to tissue. In other embodiments, there may be additional first or second patches that are also applied to tune or adjust the ratio of insulin to glucagon (or vice versa). Further developments of the invention are according to dependent claims 8-9.

The invention is also directed to a method of manufacturing a therapeutic hybrid microneedle patch, according to claim 10, including providing a mold containing a plurality of needle-shaped cavities therein; forming a mask over a portion of the mold; applying a glucagon-containing solution containing 3-(acrylamido)phenylboronic acid (APBA), 2-aminoethyl methacrylate hydrochloride (AMH), 1-vinyl-2-pyrrolidinone (VP), glucagon, crosslinker, and photoinitiator over the non-masked portion of the mold; removing the mask; crosslinking the glucagon-containing solution with ultraviolet light; applying a insulin-containing solution containing 3-(acrylamido)phenylboronic acid (APBA), 2-aminoethyl methacrylate hydrochloride (AMH), 1-vinyl-2-pyrrolidinone (VP), insulin, crosslinker, and photoinitiator over the portion of the mold that was previously masked; crosslinking the insulin-containing solution with ultraviolet light; applying a backing material over the mold and curing the backing material; and separating the therapeutic hybrid microneedle patch from the mold. Of course, the insulin-containing solution may be first applied, crosslinked, and then followed by the glucagon-containing solution. In addition, the first polymer solution may be crosslinked with the mask still in place. Different biocompatible polymer polymers may be used to form to microneedles that contain the insulin and glucagon. Further developments of the invention are according to dependent claims 11-13.

### Brief Description of the Drawings

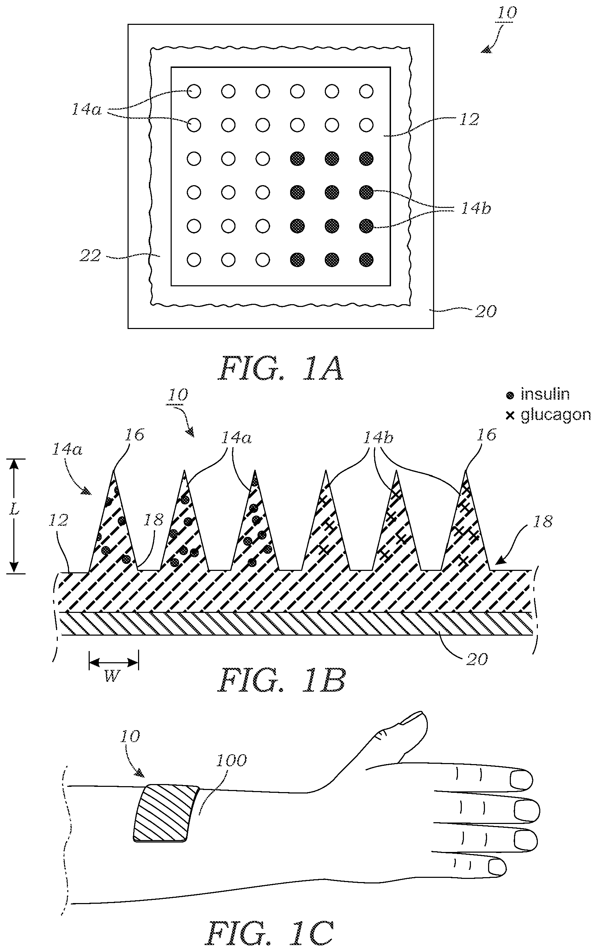

FIG. 1A illustrates a plan view of a therapeutic hybrid microneedle patch for the delivery of insulin and glucagon according to one embodiment. The therapeutic hybrid microneedle patch includes a base or substrate and a plurality of microneedles that extend from a surface thereof.
FIG. 1B illustrates a cross-sectional view of a therapeutic hybrid microneedle patch illustrating the base or substrate and the plurality of microneedles that extend from a surface thereof. Insulin and glucagon are illustrated being disposed within the microneedles.
FIG. 1C illustrates the therapeutic hybrid microneedle patch being applied to tissue of a mammal (e.g., human).

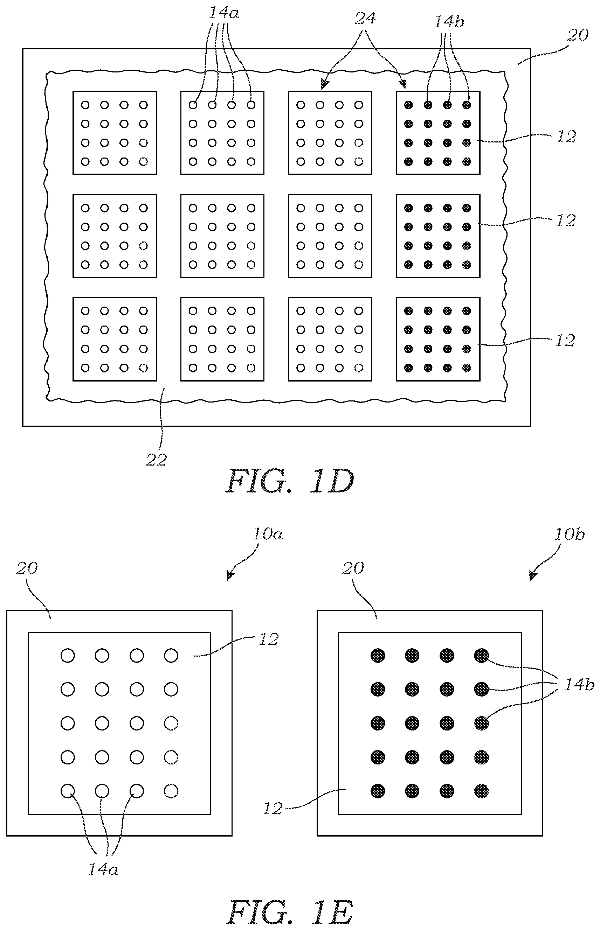

FIG. 1D illustrates a plan view of an alternative embodiment of a therapeutic hybrid microneedle patch.
FIG. 1E illustrates one embodiment of an alternative therapeutic hybrid microneedle patch system that includes a first patch that has microneedles loaded with insulin and a second patch that has microneedles loaded with glucagon.

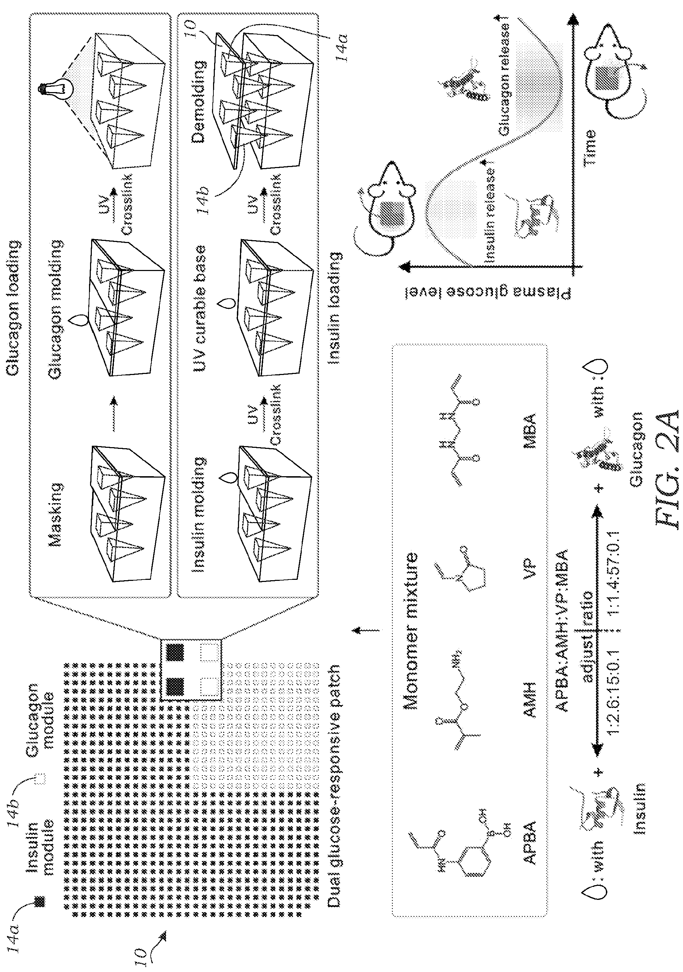

FIG. 2A is a schematic illustration of the fabrication process and glucose-responsive glycemic control mechanism of the therapeutic hybrid microneedle patch. Insulin or glucagon release can be promoted by hyperglycemic and hypoglycemic conditions, respectively. The missing microneedles at two corners of the mold are used for orientation tagging.

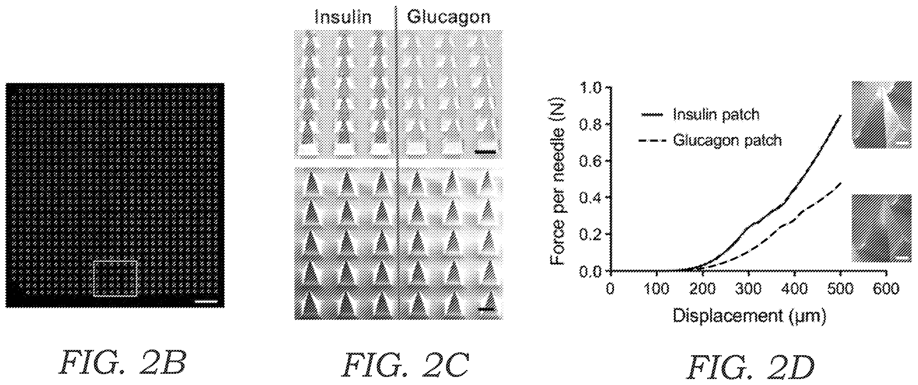

FIG. 2B is a tile-scanned fluorescence microscopy top view image of the rhodamine B-labeled glucagon (cyan) and Cy5-labeled insulin (magenta) hybrid microneedle patch. Scale bar, 2 mm.
FIG. 2C is a photographic image (top) and scanning electron microscopy image (bottom) of the therapeutic hybrid microneedle patch at the intersection. Scale bar, 300 µm.
FIG. 2D is a graph showing the mechanical performance of the glucagon and insulin microneedles, respectively. A representative enlarged microneedle SEM image is placed next to each curve. Scale bar, 100 µm.

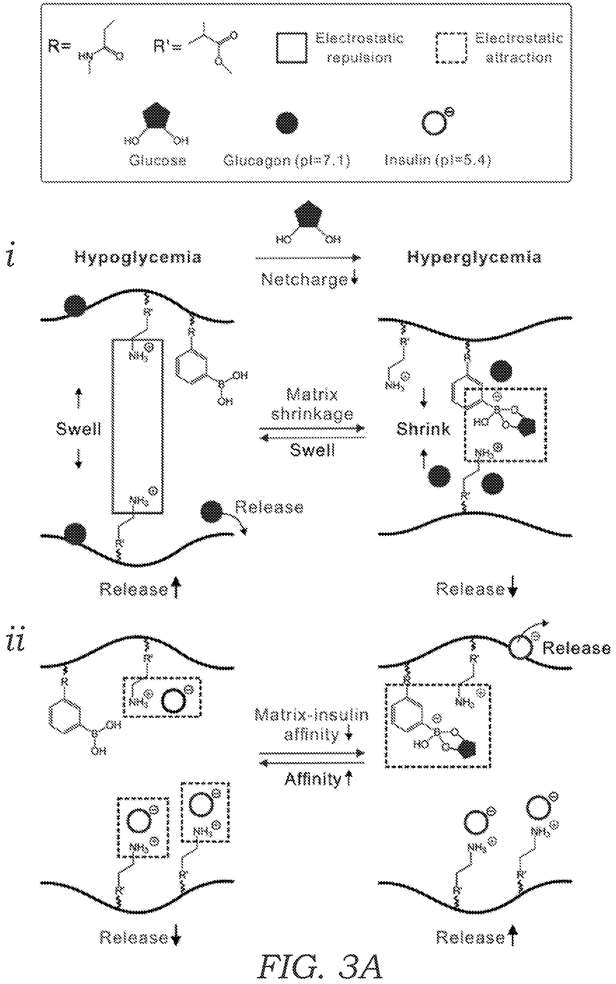

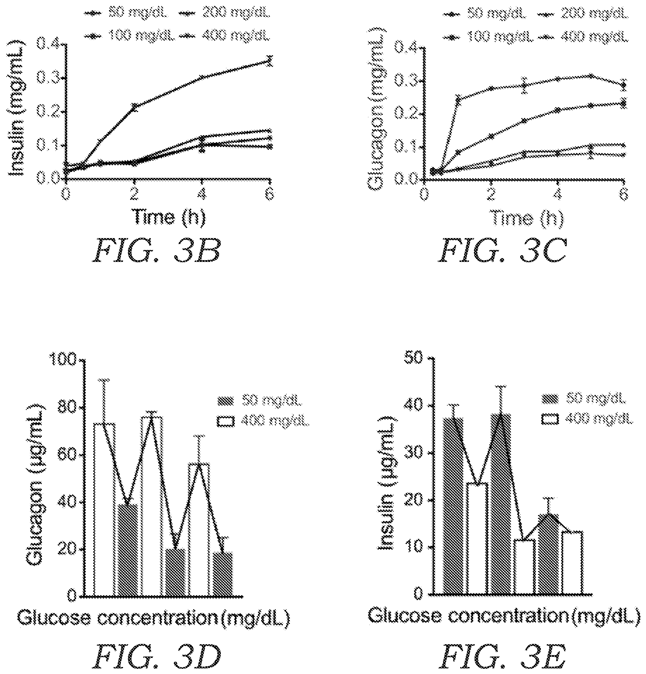

FIG. 3A illustrates the mechanism and *in vitro* evaluation of the glucose-regulated insulin/glucagon release. In hyperglycemic conditions, the formation of the glucose-boronate complexes increases the surrounding negative charges. (i) The repulsion force between the positively charged polymeric matrix is neutralized, inducing the polymer to shrink and slowing down the glucagon diffusion. (ii) The electrostatic attraction between negatively charged insulin and positively charged polymeric matrix is weakened to promote the release of insulin from the microneedles. (FIGS. 3B-3C).
FIG. 3B illustrates the *in vitro* accumulated insulin release from the glucose-responsive polymeric matrix in varying glucose concentrations at 37 °C, pH 7.4. Data points are means ± SD *(n* = 3).
FIG. 3C illustrates the *in vitro* accumulated glucagon release from the glucose-responsive polymeric matrix in varying glucose concentrations at 37 °C, pH 7.4. Data points are means ± SD *(n* = 3).
FIGS. 3D-3E illustrate the pulsatile release of insulin (FIG. 3D) and glucagon (FIG. 3E) by alternating the glucose concentrations between 50 mg/dL and 400 mg/dL. The incubation time for glucagon and insulin in each solution is 15 min and 30 min, respectively. Data points are means ± SD *(n* = 3).

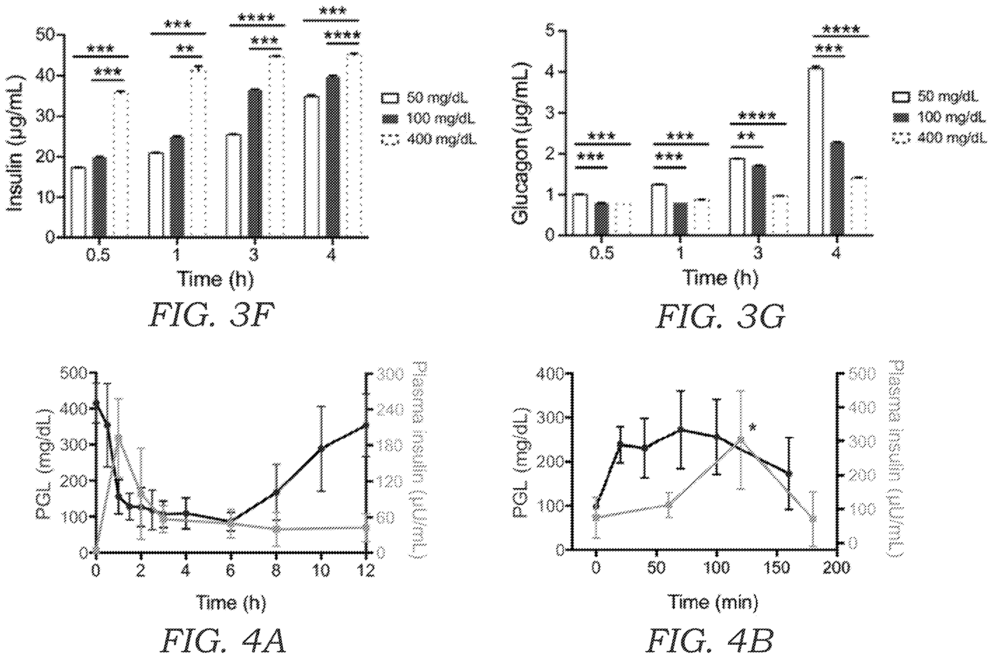

FIGS. 3F-3G illustrates the glucose-responsive release of insulin (FIG. 3F) and glucagon (FIG. 3G) from the therapeutic hybrid microneedle patch in the indicated glucose concentrations. The accumulated release is quantified with ELISA. Data points are means ± SD *(n* = 3). Statistical significance was determined by two-tailed Student's t-test. **P < 0.01, ****P* < 0.001, and ****P < 0.0001.

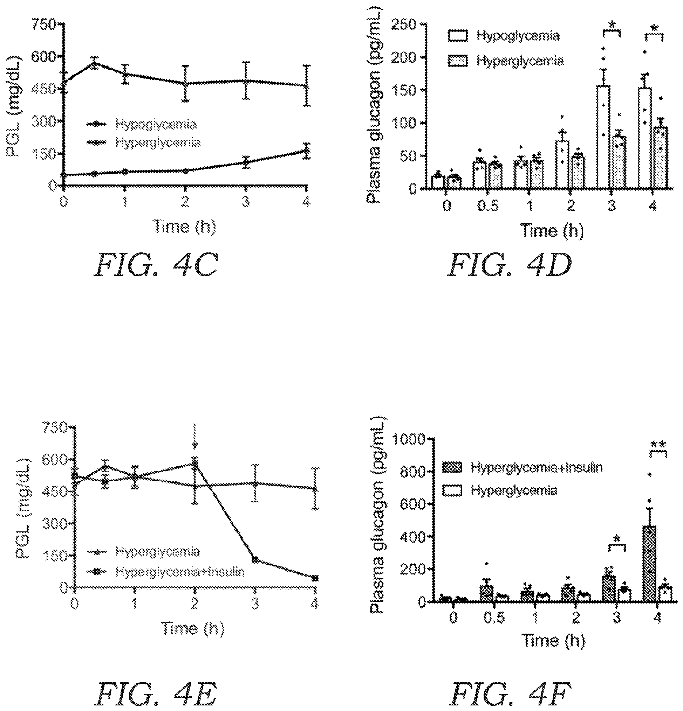

FIGS. 4A-4F illustrate the *in vivo* evaluation of the glucose-responsive insulin and glucagon release from microneedles. (FIG. 4A) PGLs and plasma human insulin concentrations in diabetic mice (*n* = 5) after insulin-only patch administration (insulin dose: 50 mg/kg). (FIG. 4B) *In vivo* glucose-responsive insulin release triggered by intraperitoneal glucose challenge at time = 0, 4 h post-administration of insulin-only patch (insulin dose: 50 mg/kg) in diabetic mice (*n* = 5). Glucose dose: 3 g/kg. (C-D) PGLs (FIG. 4C) and plasma glucagon concentrations (FIG. 4D) in diabetic mice (*n* = 5) after treated with the glucagon-only patch (glucagon dose: 17 mg/kg) in a hyperglycemic state and a hypoglycemic state (induced by overnight fasting and a subcutaneous injection of 2 U/kg insulin), respectively. Statistical significance was determined by two-tailed Student's t-test. (FIGS. 4E-4F) PGLs (FIG. 4E) and plasma glucagon concentrations (FIG. 4F) in diabetic mice (*n* = 5) treated with the glucagon-only patch (glucagon dose: 17 mg/kg) which was challenged with a subcutaneous injection of 70 U/kg insulin two hours post glucagon-only patch administration (indicated with an arrow). Statistical significance was determined by two-tailed Student's t-test. *P < 0.05, ***P* < 0.01.

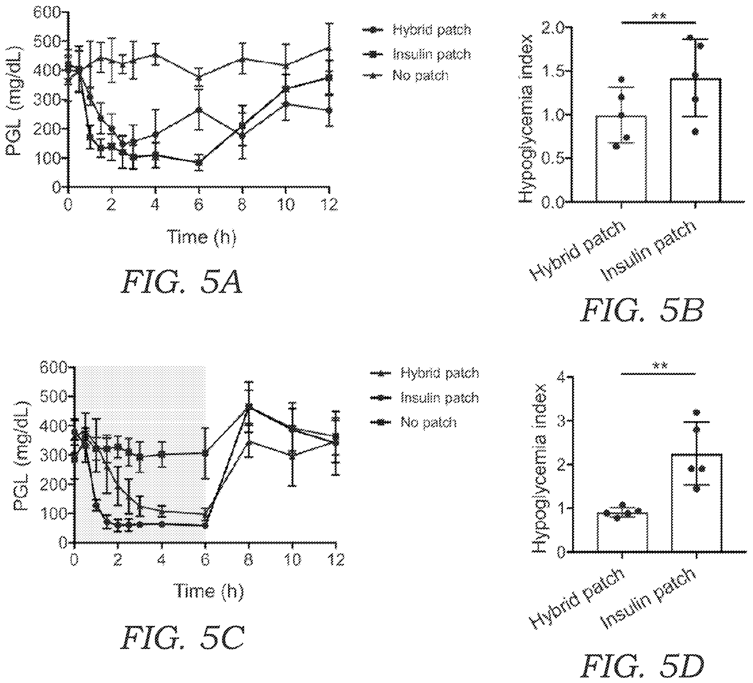

FIGS. 5A-5D illustrate the *in vivo* evaluation of the therapeutic hybrid microneedle patch safeguard effect. (FIGS. 5A-5B) PGLs (FIG. 5A) and hypoglycemia index (FIG. 5B) in diabetic mice (*n* = 5) after treatment with insulin-only patch (insulin dose: 50 mg/kg) or the therapeutic hybrid microneedle patch (insulin dose: 50 mg/kg, glucagon dose: 17 mg/kg). (FIGS. 5C-D) PGLs (FIG. 5C) and hypoglycemia index (FIG. 5D) in diabetic mice (*n* = 5) fasted for six hours (highlighted region) after treatment with insulin-only patch (insulin dose: 50 mg/kg) or the therapeutic hybrid microneedle patch (insulin dose: 50 mg/kg, glucagon dose: 17 mg/kg). Statistical significance was determined by two-tailed Student's t-test, **P < 0.01. Hypoglycemia index is defined by the difference of the initial and nadir PGL readings divided by the time spent to reach nadir PGL.

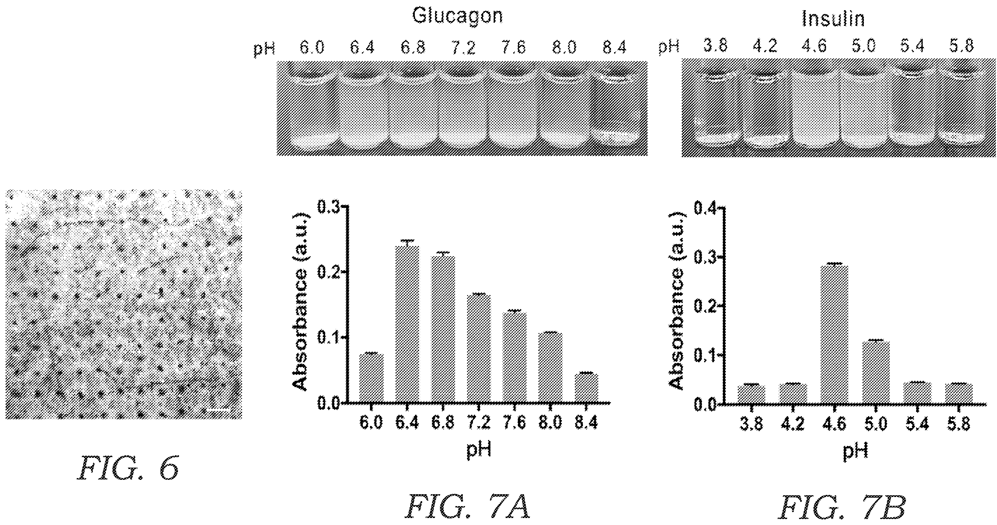

FIG. 6 illustrates an image of trypan blue stained mouse dorsum skin after transcutaneously treated with the therapeutic hybrid microneedle patch. Scale bar, 600 µm.
FIGS. 7A and 7B illustrate solubility determination images. Images (top) and absorbance at 550 nm (bottom) indicating the solubility of the glucagon (FIG. 7A) and insulin (FIG. 7B) used in the experiment. 50 µL of 20 mg/mL insulin or glucagon dissolved in 0.1 M HCl was added to the 1 mL buffer with the indicated pH value. Data points are means ± SD *(n* = 3).

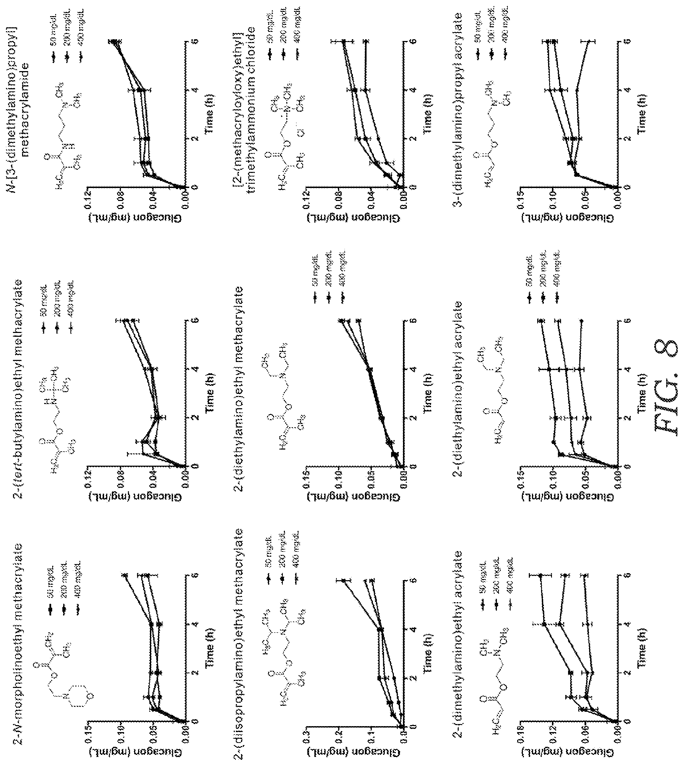

FIG. 8 illustrates the results of monomer screening (AMH/APBA ratio of 1.1). *In vitro* accumulated glucagon release from the polymeric matrix with an AMH/APBA ratio of 1.1. The releasing curve was performed in the indicated glucose concentrations at 37 °C, pH 7.4. Data are presented as mean ± SD *(n* = 3).

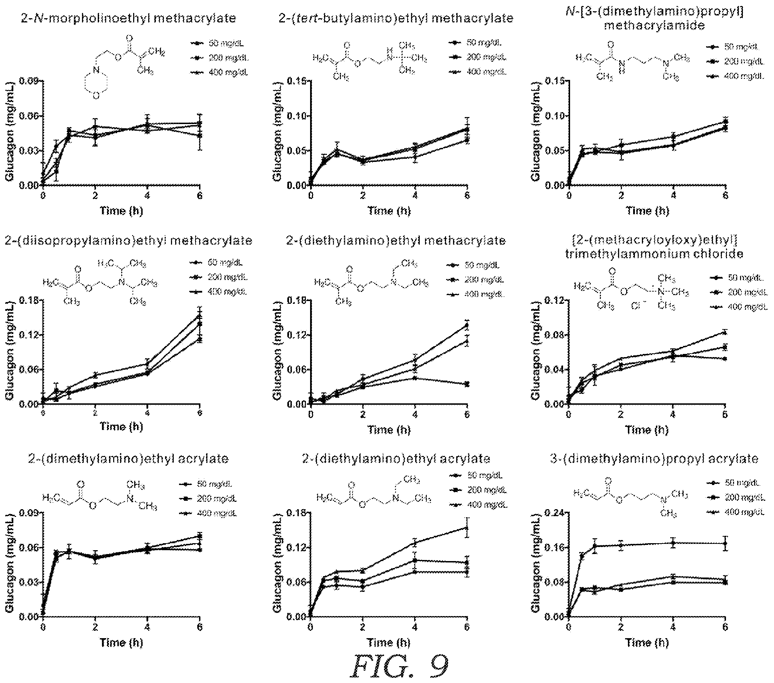

FIG. 9 illustrates the results of monomer screening (AMH/APBA ratio of 1.4). *In vitro* accumulated glucagon release from the polymeric matrix with an AMH/APBA ratio of 1.4. The releasing curve was performed in the indicated glucose concentrations at 37 °C, pH 7.4. Data are presented as mean ± SD *(n* = 3).

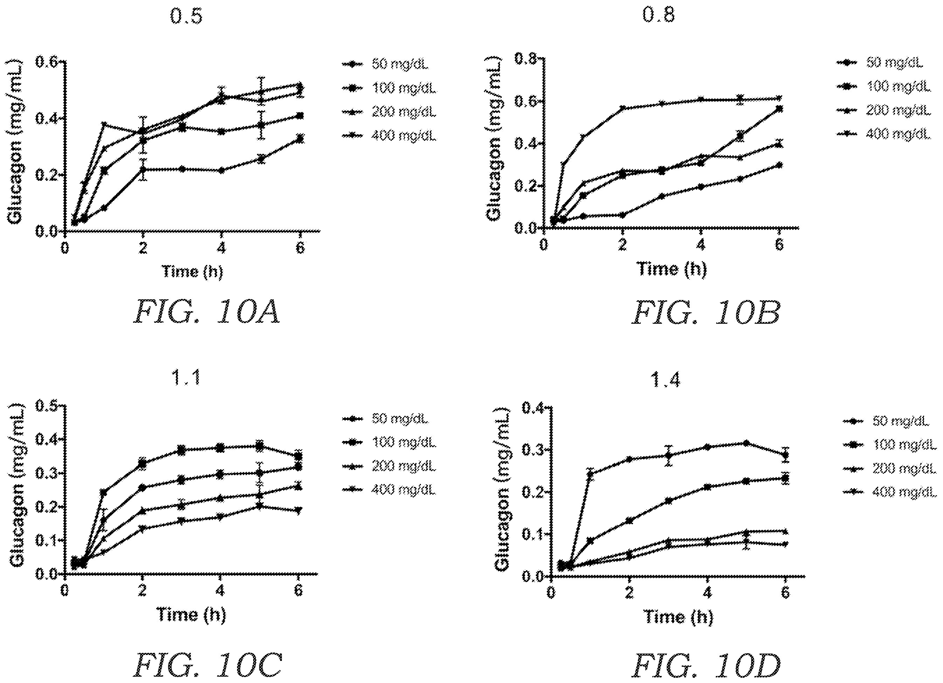

FIGS. 10A-10D illustrate the glucose-responsive glucagon performance with altered AMH and APBA ratios. (FIGS. 10A-10D) *In vitro* accumulated glucagon release from the polymeric matrix with AMH/APBA ratio of 0.5, 0.8, 1.1 and 1.4, respectively. The releasing curve was performed in the indicated glucose concentrations at 37 °C, pH 7.4. Data are presented as mean ± SD *(n* = 3).

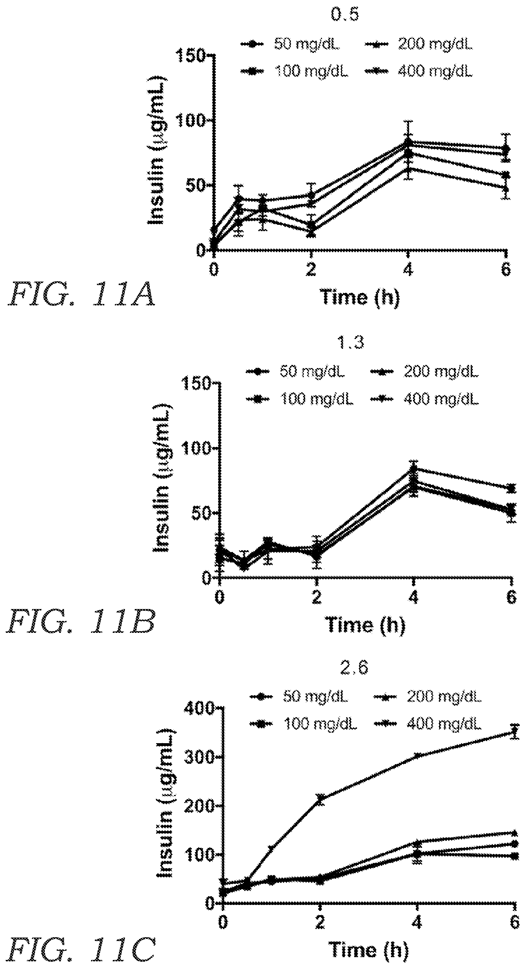

FIGS. 11A-11C illustrate the glucose-responsive insulin performance with altered AMH and APBA ratios. (FIGS. 11A-11B) *In vitro* accumulated insulin release from the polymeric matrix with an AMH/APBA ratio of 0.5 (FIG. 11A), 1.3 (FIG. 11B), and 2.6 (FIG. 11C), respectively. The releasing curve was performed in the indicated glucose concentrations at 37 °C, pH 7.4. Data are presented as mean ± SD *(n* = 3).

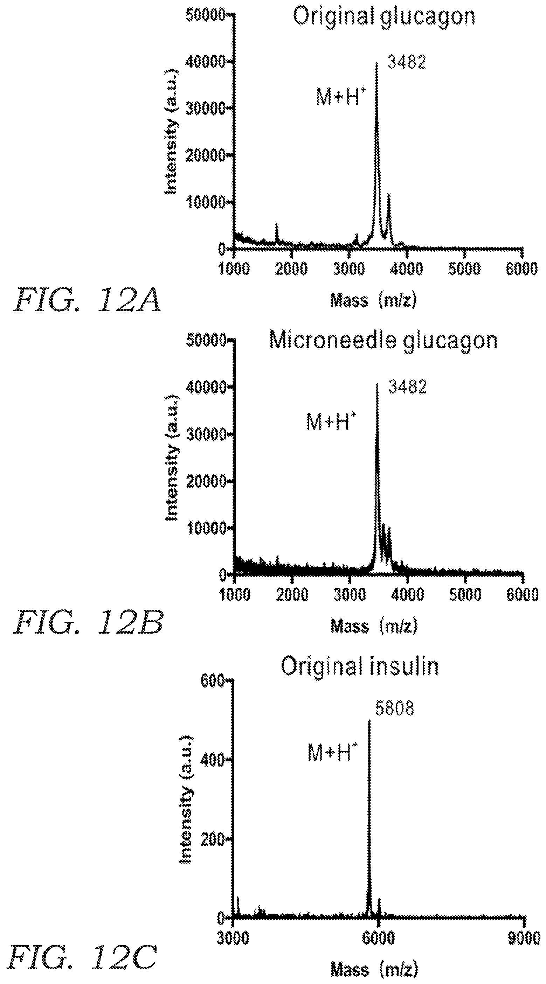

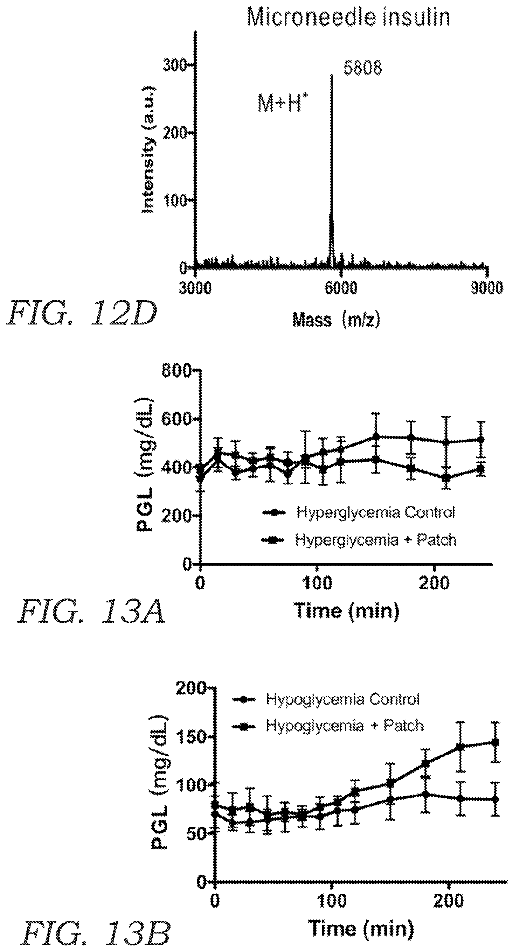

FIGS. 12A-12D illustrate matrix-assisted laser desorption ionization-time of flight (MALDI-TOF) mass spectrum. FIGS. 12A-12D show the MALDI-TOF mass spectrum of native glucagon (FIG. 12A), glucagon released from microneedles (FIG. 12B), native insulin (FIG. 12C) and insulin released from microneedles (FIG. 12D).

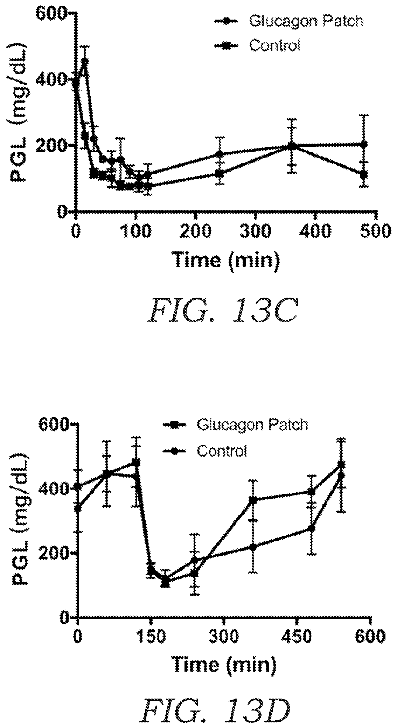

FIGS. 13A-13D illustrate the safeguard effect of the glucagon-only patch. (FIG. 13A) PGLs of diabetic mice treated with glucagon-only patch (glucagon dose: 17 mg/kg) versus control mice (no treatment). (FIG. 13B) PGLs of diabetic mice in a hypoglycemic state (induced by overnight fasting with 2 U/kg insulin injection) with and without treatment with glucagon-only patch (glucagon dose: 17 mg/kg). (FIG. 13C) PGLs of diabetic mice with and without treatment with glucagon-only patch (glucagon dose: 17 mg/kg) after injection with 70 U/kg insulin. (FIG. 13D) PGL recovery after 70 U/kg insulin injection 2 h post glucagon patch administration (glucagon dose: 17 mg/kg). Data represents mean ± SD *(n* = 5).

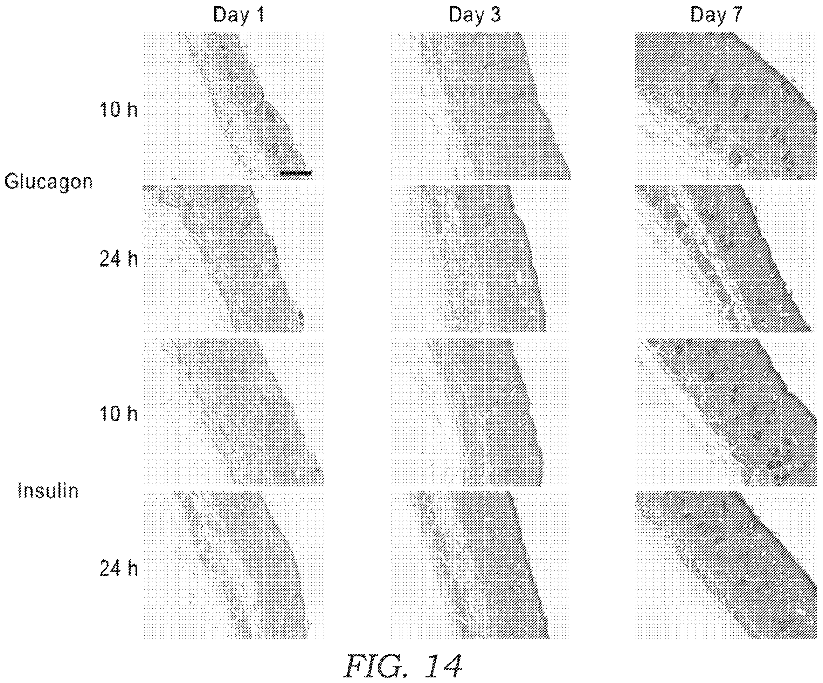

FIG. 14 illustrates images of hematoxylin and eosin (H&E) staining results. Glucagon-only patch or insulin-only patch was applied on mice skin for 10 h or 24 h, respectively. The treated parts of the skin were harvested 1-, 3-, and 7-days post microneedle removal. Neutrophil infiltration at the microneedle-treated site is insignificant one week after administration. Scale bar, 200 µm.

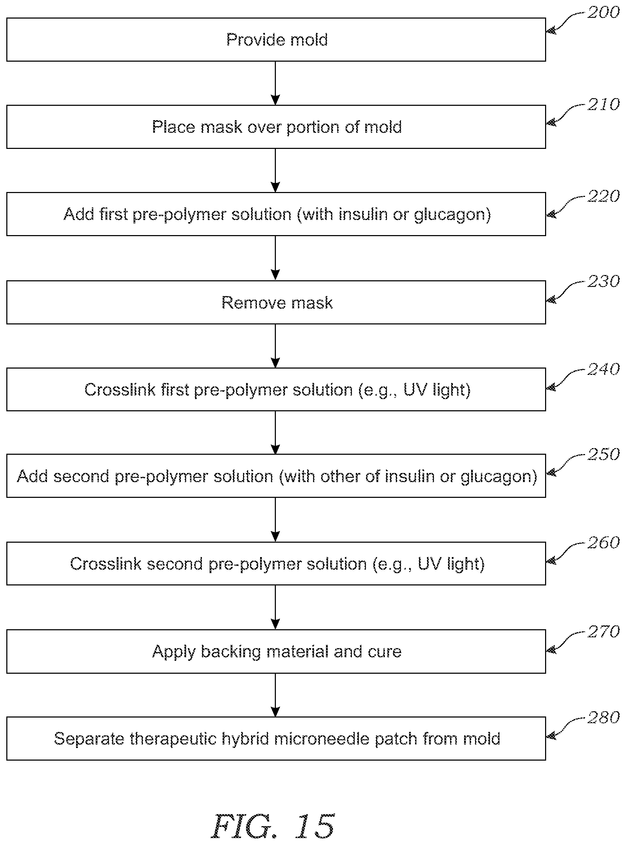

FIG. 15 illustrates the operations involved in the fabrication of the therapeutic hybrid microneedle patch according to one embodiment.

### Detailed Description of Illustrated Embodiments

FIG. 1A illustrates a plan view of a therapeutic hybrid microneedle patch 10 for the delivery of insulin and glucagon to mammalian tissue 100 (FIG. 1C) according to one embodiment. The insulin and glucagon are loaded into microneedles 14a, 14b formed in the patch 10 as described herein. The therapeutic hybrid microneedle patch 10 is made from a biocompatible polymer such as is described herein. The therapeutic hybrid microneedle patch 10 may in some embodiments be partly or entirely biodegradable. The term biodegradable in the context of a therapeutic hybrid microneedle patch 10 refers to the base or substrate 12 and/or the microneedles 14a, 14b being formed from a material that is at least partially biodegradable. In other embodiments, such as the specific biocompatible polymer system described in the experiments herein, the therapeutic hybrid microneedle patch 10 is applied to tissue 100 and remains while the insulin and glucagon are released. The therapeutic hybrid microneedle patch 10 is then removed from the tissue after a period of time. This may be hours or several/many days after initial application of the therapeutic hybrid microneedle patch 10.

In one embodiment, some microneedles 14a within a single therapeutic hybrid microneedle patch 10 are loaded with insulin while other microneedles 14b within the same therapeutic hybrid microneedle patch 10 are loaded with glucagon. The ratio of insulin to glucagon (or glucagon to insulin) within the therapeutic hybrid microneedle patch 10 may be adjusted or tuned by altering the number of microneedles 14a, 14b that are loaded with insulin and/or glucagon. In one embodiment, the therapeutic hybrid microneedle patch 10 contains more insulin than glucagon. For example, the number of microneedles 14a in the therapeutic hybrid microneedle patch 10 that contain insulin may be a majority of the total microneedles 14a, 14b in the therapeutic hybrid microneedle patch 10. Thus, in this example, more than 50% of the total microneedles 14a, 14b in the therapeutic hybrid microneedle patch 10 are loaded with insulin (i.e., microneedles 14a). The remaining microneedles 14b may be loaded with glucagon. In another embodiment, about 75% of the microneedles 14a are loaded with insulin while about 25% of the microneedles 14b are loaded with glucagon. It should be appreciated that the relative amount of insulin and glucagon in the therapeutic hybrid microneedle patch 10 may be adjusted or tuned as needed (e.g., insulin:glucagon ratio of 50.1: 49.9, 55:45, 60:40, 65:35, 70:30, 75:25, 80:20 and points therebetween). While the relative amounts of insulin and glucagon in the therapeutic hybrid microneedle patch 10 may be adjusted through the respective numbers of microneedles 14a, 14a in the therapeutic hybrid microneedle patch 10 other methods of controlling the relative amounts of each (e.g., ratio) may be used. For example, the loading capacity of certain microneedles 14a, 14b may be made larger to accommodate larger loading of insulin and/or glucagon. For example, certain microneedles 14a, 14b may be larger than other microneedles 14a, 14b to increase loading capacity. In such an embodiment, the relative amounts of insulin and/or glucagon can be adjusted without having to alter the number of microneedles 14a, 14b.

In one embodiment, the therapeutic hybrid microneedle patch 10 may be applied to live mammalian tissue 100 for the treatment of hyperglycemic and hypoglycemic conditions in the mammalian subject. In one specific embodiment, the therapeutic hybrid microneedle patch 10 is used to treat diabetes. The mammalian tissue 100 may include any tissue but skin tissue 100 is contemplated as being the most appropriate. The therapeutic hybrid microneedle patch 10 is thus a transdermal therapeutic treatment.

The therapeutic hybrid microneedle patch 10 includes a base or substrate 12 that includes a plurality of microneedles 14a, 14b that extend or project from the substrate 12. The plurality of microneedles 14a, 14b generally extend or project in a perpendicular direction from a surface of the base or substrate 12 (seen in FIG. 1B). The plurality of microneedles 14a, 14b may be arranged in a regular repeating array as illustrated in FIG. 1A or, alternatively, they may be arranged in a random pattern. In one embodiment, the plurality of microneedles 14a, 14b that are formed on the base or substrate 12 may have substantially similar shapes and sizes. However, in other embodiments, the plurality of microneedles 14a, 14b may have different shapes and/or sizes. For example, the perimeter region of the array or field of microneedles 14a, 14b that extend from the base or substrate 12 may be longer or have different sizes and/or shapes than those in the central region of the therapeutic hybrid microneedle patch 10 to better secure the therapeutic hybrid microneedle patch 10 to site of application. Likewise, in other embodiments, the microneedles 14a containing insulin may have different sizes and/or shapes (or dimensions) than the microneedles 14b containing glucagon.

In one particular embodiment, the microneedles 14a, 14b, as their name implies, have a needle-like shape. For example, the microneedles 14a, 14b may include a sharpened tip 16 (seen in FIG. 1B) that aid in penetrating the tissue 100 (seen in FIG. 1C). The length (L) of the microneedles 14 may vary although typically the microneedles 14a, 14b extend less than about 2.0 mm from the base or substrate 12 (FIG. 1B). A typical length of the microneedles 14 is around 500-1,500 µm, although the dimensions may extend outside this range. The base 18 of the microneedle 14a, 14b is wider than the tip 16. Typically, the base 18 of the microneedle 14a, 14b may have a diameter or width (W) that is less than about 600 µm (e.g., 300 µm base width and a height of around 700 µm) (FIG. 1B). The microneedles 14a, 14b may be separated from one another by a pitch or spacing that may be several hundred µm (e.g., 200 µm). The particular numbers, density, dimensions, and shape(s) of the microneedles 14a, 14b are controlled by the particular construction of the mold that is used to form the therapeutic hybrid microneedle patch 10, which is described more in detail below. As described herein, the microneedles 14a, 14b are pyramid shaped (e.g., pyramidal), although other shapes are contemplated (e.g., conical). The base 12 of the therapeutic hybrid microneedle patch 10 may include a flexible material when hydrated or swollen. The number and configuration of the microneedles 14a, 14b may vary depending on the subject. For example, a larger mammal may require a larger therapeutic hybrid microneedle patch 10 with a larger number of microneedles 14a, 14b. In the tested therapeutic hybrid microneedle patch 10, a 30 x 30 array of microneedles 14a, 14b was used.

Still referring to FIGS. 1A and 1B, the base or substrate 12 which holds the microneedles 14a, 14b may be optionally bonded or otherwise adhered to a backing material 20 (e.g., through the use of an adhesive, chemical linking, or the like). The backing material 20 may be made from a woven fabric, a plastic material such as polyvinylchloride, polyethylene, or polyurethane, or latex. The backing material 20 may be flexible so that the therapeutic hybrid microneedle patch 10, when applied, can conformally cover the tissue 100 (seen in FIG. 1C). Optionally, the backing material 20 may include an adhesive material 22 that covers all or a portion of the tissue-facing surface of the backing material 20. For example, adhesive may be formed on the backing material 20 around the periphery of the base or substrate 12 or the backing material 20 so that the base or substrate 12 may be secured in place to the surface of the tissue 100. The adhesive material 22 aids in securing the therapeutic hybrid microneedle patch 10 to the tissue 100. The adhesive material 22 may include resins (e.g., vinyl resins), acrylates such as methacrylates epoxy diacrylates. In other embodiments, the backing material 20 may be omitted entirely. Alternatively, the therapeutic hybrid microneedle patch 10 may be secured to the tissue 100 via engagement of the microneedles 14 in the tissue 100. In still other alternatives, the therapeutic hybrid microneedle patch 10 may be secured to the tissue 100 using an adhesive or glue located on the base or substrate 12. A separate bandage or wrap may also be used to secure the therapeutic hybrid microneedle patch 10 to the tissue 100.

In one alternative embodiment which is illustrated in FIG. 1D, multiple sub-patches 24 may be integrated into the backing material 20 to make the final therapeutic hybrid microneedle patch 10. This may be useful for large coverage areas or curved surfaces that may pose a risk of breakage to the base or substrate 12. The various sub-patches 24, while having some rigidity, are still able to conform to the surface of the tissue 100 (e.g., FIG. 1C) due the flexible backing material 20 which enables bending of the overall therapeutic hybrid microneedle patch 10. Because individual sub-patches 24 are smaller in size these do not experience significant bending stresses which would otherwise cause a larger, rigid structure to break in response to bending and/or manipulation. Bending or flexing can occur within the backing material 20 between the locations of where the sub-patches 24 are located (e.g., between the rows and columns of sub-patches 24). In this embodiment, certain sub-patches 24 may include microneedles 14a loaded with insulin while other sub-patches 24 may include microneedles 14b loaded with glucagon (illustrated in FIG. 1D).

As yet another alternative and with reference to FIG. 1E, multiple therapeutic hybrid microneedle patches 10 are used. In this embodiment, a first therapeutic hybrid microneedle patch 10a is provided that has microneedles 14a that contain insulin therein. A second therapeutic hybrid microneedle patch 10b is provided that has microneedles 14b that contain glucagon therein. Both therapeutic hybrid microneedle patches 10a, 10b are applied to the tissue 100 to achieve the same or similar results to a single patch 10 that has microneedles 14a, 14b loaded with both insulin and glucagon. The particular ratio or relative composition of insulin and glucagon (such as the examples described herein in the context of a single therapeutic hybrid microneedle patch 10) can be adjusted by adjusting, for example, the numbers, sizes, and shapes of the microneedles 14a, 14b of the two therapeutic hybrid microneedle patches 10a, 10b. The therapeutic hybrid microneedle patches 10a, 10b may be co-located at the same region of tissue 100 or they may be placed in different locations on the tissue 100. While this alternative embodiment uses two therapeutic hybrid microneedle patches 10 it should be understood that additional numbers of therapeutic hybrid microneedle patches 10 may be used. For example, there could be a single therapeutic hybrid microneedle patch 10a that contains glucagon in the microneedles 14a and two (or more) therapeutic hybrid microneedle patches 10a that contain insulin in the microneedles 14b. The patients or subjects that are being administered the therapeutic hybrid microneedle patches 10a, 10b may be given a prescription or instructions to, for example, apply one type of therapeutic hybrid microneedle patch 10a in combination with one or more other types of therapeutic hybrid microneedle patch 10b. Of course, incorporating both types of microneedles 14a, 14b into a single therapeutic hybrid microneedle patch 10 prevents patients or caregivers from accidentally deviating from the desired ratio of insulin to glucagon since only a single therapeutic hybrid microneedle patch 10 is applied.

The microneedles 14a, 14b of the therapeutic hybrid microneedle patch 10 is fabricated by copolymerization of the monomers of 3-(acrylamido)phenylboronic acid (APBA), 2-aminoethyl methacrylate hydrochloride (AMH), and 1-vinyl-2-pyrrolidinone (VP) but contain either insulin or glucagon. A mask-mediated sequential photo-polymerization preparation method as described herein facilitates the integration of the two types of microneedles 14a, 14b, loaded with insulin and glucagon, respectively, in the therapeutic hybrid microneedle patch 10 which mimics islet cell secretion of insulin or glucagon in response to the plasma glucose levels (PGLs). The composition ratio of insulin and glucagon in the therapeutic hybrid microneedle patch 10 can be easily adjusted by arranging the loading pattern of the microneedles 14a, 14b.

To form the therapeutic hybrid microneedle patch 10 for delivering insulin and glucagon to living tissue, a molding process is used. FIG. 15 illustrates the operations to fabricate the therapeutic hybrid microneedle patch 10 according to one embodiment. In operation 200, a mold (for example, made from silicon) is provided that contains a plurality of needle-shaped cavities therein. The mold may be made from a number of materials including silicon, metal, glass, or the like. Next, as seen in operation 210, a mask is placed over a portion of the mold. The region(s) of the mold that is covered by the mask is blocked from the liquid pre-polymer solution from entering the needle-shaped cavities. The mask may be configured to have dedicated regions of the therapeutic hybrid microneedle patch 10 contain insulin-containing microneedles 14a and glucagon-containing microneedles 14b or they may be interspersed with one another.

Next, as seen in operation 220, a pre-polymer solution is applied to the mold. In one embodiment, this pre-polymer solution includes a glucagon-containing solution containing 3-(acrylamido)phenylboronic acid (APBA), 2-aminoethyl methacrylate hydrochloride (AMH), 1-vinyl-2-pyrrolidinone (VP), glucagon, crosslinker, and a photoinitiator. This is applied to mold where the fluid enters the needle-shaped cavities in the non-masked portion of the mold. In operation 230, the mask is removed and in operation 240, the glucagon-containing solution that fills a first plurality of needle-shaped cavities is then subject to ultraviolet light to initiate crosslinking. Note that mask may be removed after the crosslinking operation or before. After this first crosslinking operation, a second pre-polymer solution is applied to the mold to fill the remaining second plurality of needle-shaped cavities. This is illustrated in operation 250 of FIG. 15. In one embodiment, this pre-polymer solution includes an insulin-containing solution containing 3-(acrylamido)phenylboronic acid (APBA), 2-aminoethyl methacrylate hydrochloride (AMH), 1-vinyl-2-pyrrolidinone (VP), insulin, crosslinker, and a photoinitiator over the portion of the mold that was previously masked. In operation 260, the insulin-containing solution is then subject to ultraviolet light to initiate crosslinking of the second pre-polymer solution. Next, in operation 270, a backing material is applied over the mold and cured. After curing, the now-formed therapeutic hybrid microneedle patch 10 is then separated from the mold as seen in operation 280. The therapeutic hybrid microneedle patch 10 may be maintained in this "dry" state until use.

While the specific therapeutic hybrid microneedle patch 10 formed in the mold was crosslinked using ultraviolet light it should be appreciated that other polymer materials may use other light wavelengths to initiate crosslinking of the polymer material. In addition, some alternative embodiments may omit light altogether and initiate crosslinking using a crosslinking agent that is added to the polymer precursor material (e.g., pre-polymer solution) just prior to placement on the mold. In still other embodiments, an external stimulus such as heat may be used to initiated the crosslinking process.

To use the therapeutic hybrid microneedle patch 10, the therapeutic hybrid microneedle patch 10 is applied to the tissue 100. In one example, the therapeutic hybrid microneedle patch 10 is manually pressed with firm pressure into the tissue 100 (e.g., skin) so that the microneedles 14a, 14b penetrate into the tissue. An applicator such as a stamping tool, roller, or the like may also be used to apply the therapeutic hybrid microneedle patch 10. The therapeutic hybrid microneedle patch 10 may adhere to the tissue by mechanical adherence, through the use of an adhesive, or through a bandage, wrap, or the like that can secured the therapeutic hybrid microneedle patch 10 to the tissue. The therapeutic hybrid microneedle patch 10 remains in place for a period of time which may include several hours or several/many days. After the elapsed time or after the therapeutic hybrid microneedle patch 10 no longer functions as desired the therapeutic hybrid microneedle patch 10 is removed from the tissue 100. The user or healthcare provider may physically remove the therapeutic hybrid microneedle patch 10 from the tissue. In embodiments that use multiple therapeutic hybrid microneedle patches 10a, 10b, the multiple therapeutic hybrid microneedle patches 10a, 10b are applied and removed in the same manner.

### Experimental

A therapeutic hybrid microneedle patch 10 is disclosed that can deliver insulin and glucagon in a glucose-dependent manner. The therapeutic hybrid microneedle patch 10 is constructed of regions of insulin loaded microneedles 14a or glucagon loaded microneedles 14b, thereby mimicking the functionality of pancreatic islet cells for the comprehensive regulation of blood glucose levels. The microneedles 14a, 14b are copolymerized from the same monomers of 3-(acrylamido)phenylboronic acid (APBA), 2-aminoethyl methacrylate hydrochloride (AMH), and 1-vinyl-2-pyrrolidinone (VP) but contain different ratios of each monomer (FIG. 2A). A mask-mediated sequential photo-polymerization preparation strategy as described above facilitates the integration of the two types of microneedles 14a, 14b, loaded with insulin and glucagon, respectively, into a therapeutic hybrid microneedle patch 10 which mimics islet cell secretion of insulin or glucagon in response to the plasma glucose levels (PGLs). The composition ratio of the therapeutic hybrid microneedle patch 10 can be easily adjusted by arranging the loading pattern of the microneedles 14a, 14b (or through the use of multiple therapeutic hybrid microneedle patches 10a, 10b in other embodiments). The human islet composes of 50-60% of *β*-cells and 30-45% of *α*-cells. Similarly, in one particular implementation or embodiment one-quarter of the microneedles 14b in the therapeutic hybrid microneedle patch 10 is loaded with glucagon, while the remaining three-quarters microneedles 14a are loaded with insulin.

The microneedles 14a, 14b of the therapeutic hybrid microneedle patch 10 comprise insulin- and glucagon-loaded polymeric matrix. To distribute the glucagon formulation into one-quarter of the microneedles 14b, a polyvinylpyrrolidone (PVP) microneedle "mask" was used to prevent liquid infiltration into the insulin microneedles 14a of the microneedle mold. A glucagon-preloaded (7 wt.%) monomer mixture of VP, AMH, APBA, photoinitiator, and crosslinker was then added to the mold followed by vacuum and photo-polymerization on ice. After solidification of the glucagon-loaded microneedles 14b, the mask was peeled off and the insulin-preloaded (7 wt.%) mixture, containing the same monomer components but in altered ratios, was added to the mold and underwent the same vacuum and photo-polymerization process. The formed pyramid-shaped microneedles 14a, 14b with a width of 300 µm at the base and a height of 700 µm were arranged in a 30 × 30 array. To give the therapeutic hybrid microneedle patch 10 a transparent flexible base or substrate 12, Norland Optical Adhesive 86 was used, a commercial ultraviolet-curable material, on top of the microneedles 14a, 14b for demolding. The fluorescence image of the therapeutic hybrid microneedle patch 10 revealed that rhodamine B-labelled glucagon needles and Cy5-labeled insulin needles were successfully separated into the predesigned pattern (FIG. 2B). The magnified photo and SEM image (FIG. 2C) further confirmed the successful integration of the two types of microneedles 14a, 14b with a negligible difference in surface morphology. Additionally, the fracture force of the insulin and glucagon microneedles 14a, 14b was measured and found to be comparable at 0.26 N and 0.28 N per microneedle, respectively (FIG. 2D), both sufficient for skin penetration (FIG. 6).

The glucose-responsive mechanism of insulin and glucagon delivery can be attributed to the synergistic net charge shift of the AMH/APBA polymeric network at various glucose concentrations, the difference in isoelectric points (pIs) of insulin and glucagon at physiological pH, and the consequent shrinkage or swelling of the surrounding polymeric gel matrix (FIG. 3A). Insulin has an isoelectric point around pH 5.4 and therefore exhibits a negative charge and high solubility at physiological pH. By contrast, glucagon has low solubility in the pH range of 6 to 8 due to its isoelectric point of approximately 7.1 (see FIGS. 7A & 7B). The APBA monomer can reversibly bind with glucose to generate negatively charged cyclic boronate esters. Meanwhile, the positively charged AMH monomer is used to adjust the charge of the polymeric matrix; this monomer outperformed nine other positively charged monomers partnered with APBA at the ratios of 1.1 and 1.4 (FIGS. 8 and 9). The glucose-responsive release of insulin or glucagon is determined by manipulating the ratios of the major monomer components (FIGS. 10A-10D and 11A-11C), with the AMH to APBA ratio of 1.4 for the glucagon needles 14b and 2.6 for the insulin needles 14a. When conditions are changed from normoglycemia to hyperglycemia, a more negatively charged glucose-boronate complex is formed. The decrease in net positive charge weakens the electrostatic attraction between insulin and the positively charged matrix, promoting the release of the insulin molecules from the insulin microneedles 14a. For the glucagon matrix, increased negative charge neutralizes the initially positively charged matrix, which contracts the polymeric network and prohibits the diffusion of the glucagon molecules from the glucagon microneedles 14b. Together, promoted insulin release and limited glucagon release were observed at a high glucose level of 400 mg/dL (FIGS. 3B and 3C). When surrounding conditions are hypoglycemic, the insulin matrix restores the initial net charge, slowing down insulin release, while the increased positive net charge generate repulsive interactions in the matrix, freeing the glucagon molecules from the polymeric network. This glucagon release mechanism was verified by recording the release profile of the polymeric matrix embedded with AMH to APBA molar ratios of 0.5, 0.8, and 1.1, where reversing the amount of AMH and APBA resulted in an opposite pattern of glucose-responsive behavior (FIGS. 10A-10D).

The pulsatile release profiles for the insulin and glucagon formulations, respectively were characterized and demonstrated several cycles of glucose-responsive hormone release by alternating incubation of the polymeric matrix in hypoglycemic (50 mg/dL) and hyperglycemic solutions (400 mg/dL) (FIG. 3D and 3E). To assess the glucose-responsive behavior of the integrated insulin and glucagon formulations, hormone levels in varying concentrations of glucose were quantified by the enzyme-linked immunosorbent assay (ELISA). As shown in FIGS. 3F-3G, the glucose-dependent release performance was similar to the individual release profile. The release rate of insulin or glucagon from their corresponding microneedle 14a, 14b was regulated in a glucose-dependent but counterregulatory manner. Additionally, matrix-assisted laser desorption ionization-time of flight (MALDI-TOF) mass spectrum analysis of the native insulin/glucagon and the insulin/glucagon released from the microneedles 14a, 14b confirmed that the protein molecules stayed intact throughout the polymerization process (FIGS. 12A-12D).

Next, the *in vivo* glycemic regulation abilities of the respective insulin-only and glucagon-only patches were assessed in a streptozotocin (STZ)-induced insulin-deficient diabetic mouse model. PGLs in mice treated with the insulin patch (dose: 50 mg/kg) approached a normoglycemia level (< 200 mg/dL) within one hour and stayed in this range for up to six hours. The plasma insulin level reached a peak at one hour and was stabilized after three hours (FIG. 4A). *In vivo* glucose responsiveness was assessed with an intraperitoneal glucose tolerance test (IPGTT) performed four hours posttreatment at a dosage of 3.0 g/kg. A spike in PGLs was observed, followed by a decreasing trend in PGLs over the following period of observation (FIG. 4B). In response to the initial increase in PGLs, a significant spike of plasma insulin (FIG. 4B) was recorded within one hour of the PGL spike.

The release profile of the glucagon-only patch on two groups of diabetic mice were characterized: one with hyperglycemic PGLs (untreated) and one with hypoglycemic PGLs (treated with overnight fasting and a subcutaneous injection of 2 U/kg insulin). Simultaneous study of the PGLs and plasma glucagon level showed a notably higher plasma glucagon level in the group with hypoglycemia three hours post-administration. A subsequent increase of PGLs to the normal range was also achieved (FIGS. 4C and 4D). An intraperitoneal insulin tolerance test (IPITT) was performed by administering a subcutaneous injection of 70 U/kg insulin two hours post glucagon-only patch administration, after which PGLs of the insulin-challenged group decreased and reached hypoglycemia in two hours (FIG. 4E). A gradual increase in the plasma glucagon level was observed in response to the drop in PGLs. By contrast, the control diabetic mice group without the insulin injection showed no fluctuation in the release rate of glucagon and therefore, did not cause a further rise in PGL (FIG. 4F and FIG. 13A). Taken together, both the insulin-only patch and glucagon-only patch displayed glucose-regulatory release to maintain normoglycemia.

To substantiate the capability of the glucagon microneedles 14b to mitigate hypoglycemia, the diabetic mice in hypoglycemic conditions (induced by overnight fasting with a subcutaneous injection of 2 U/kg insulin) were treated with the glucagon-only patch and kept under fasting conditions during the treatment period. The patch-treated mice restored normoglycemic conditions after two hours, while the non-patch-treated group remained in hypoglycemic ranges (FIG. 13B). To further characterize the safeguard capability of the glucagon patch, the diabetic mice were deprived of food throughout the experiment and subjected to an insulin injection (70 U/kg). The glucagon-only patch slowed down the decrease in PGLs after the insulin injection and ultimately prevented PGLs from dropping below 50 mg/dL (FIG. 13C). In contrast, the PGLs of the control group rapidly declined to hypoglycemic ranges where they remained for two hours. When the diabetic mice under normal food intake conditions were challenged with an insulin injection (70 U/kg), the patch-treated group still experienced a faster PGL recovering rate compared with the control group (FIG. 13D).

The therapeutic hybrid microneedle patch 10 was then administered and the glucose-responsive treatment performance was compared with the performance of the separate insulin-only patch. As expected, the integration of the glucagon microneedles 14b with the insulin microneedles 14a delayed the decrease of the PGLs, and a fluctuating pattern in PGLs around the normoglycemic range was observed in the therapeutic hybrid microneedle patch-treated group. Compared to the flat curve of the insulin-only patch (FIG. 5A), this result validates the alternating regulatory effect between the insulin and glucagon microneedles 14a, 14b. Additionally, the therapeutic hybrid microneedle patch 10 showed a remarkably reduced hypoglycemic index (defined by the difference of the initial and nadir PGL readings divided by the time spent to reach nadir) average of 0.99, compared with 1.42 of the insulin-loaded patch (FIG. 5B). The effect of a simulated delayed meal intake on the performance of the insulin-only and therapeutic hybrid microneedle patch 10 was characterized by subjecting the diabetic mice to six hours of fasting. The therapeutic hybrid microneedle patch 10 regulated the PGLs within a normoglycemic range, while the insulin-only patch led to hypoglycemia (FIG. 5C). This notable safeguard effect for meal delay was also reflected in the maintained hypoglycemia index average of 0.91 for the therapeutic hybrid microneedle patch 10 and increased hypoglycemia index average of 2.25 for the insulin-only patch (FIG. 5D).

Regarding biocompatibility, a matrix-crosslinked and removable therapeutic hybrid microneedle patch 10 may eliminate post-treatment safety issues associated with dissolvable matrixes or implantable devices. Hematoxylin and eosin staining (H&E) of the mice skin treated with each patch for 10 hours or 24 hours was evaluated, respectively, and for application as long as 24 hours, insignificant neutrophil infiltration at the administration sites was shown one week after administration (FIG. 14).

In summary, a dual glucose-responsive insulin and glucagon delivery device or therapeutic hybrid microneedle patch 10 has been described which functions as an external "pancreatic islet" across a spectrum of glucose ranges. Treatment with this hybrid formulation may be particularly beneficial for individuals with diabetes in the setting of lifestyle changes, irregular schedules and missed meals, or inaccurate insulin dosing. Opportunities remain for the therapeutic hybrid microneedle patch 10 or patch system (from multiple such therapeutic hybrid microneedle patches 10a, 10b) to seek for enhanced release kinetics and prolonged glycemic control through optimization of formulation and microneedle design. To facilitate future translation of the therapeutic hybrid microneedle patch 10 to a wide range of users, the ratio of the insulin and glucagon microneedles 14a, 14b can be customized during the fabrication procedure to fulfill the diverse needs of different individuals. Moreover, 3D printing technologies could automate the design procedure and equip the therapeutic hybrid microneedle patch 10 with a stamp-like applicator to standardize the skin penetration process. Finally, the masking and sequential photo-polymerization approach employed in making the therapeutic hybrid microneedle patch 10 may be expanded to other drug delivery applications for co-delivering multiple therapeutics with enhanced efficacy and safety.

### Materials and Methods

### Materials

All chemicals were purchased from Sigma-Aldrich unless otherwise specified and were used as received. Norland Optical Adhesive 86 was purchased from Norland Products Inc. 3-(acrylamido)phenylboronic acid was purchased from Boron Molecular (catalog number BM1195). Human recombinant insulin was purchased from Thermo Fisher Scientific (catalog number A11382IJ). Glucagon and 2-aminoethyl methacrylate hydrochloride were purchased from Fisher Scientific (catalog number 50-751-6116, 50-145-4119).

### Synthesis of therapeutic hybrid microneedle patch

The therapeutic hybrid microneedle patch 10 was prepared by masking-assisted sequential polymerization under ultraviolet irradiation. First, glucagon (7 wt.%) is preloaded in the VP monomer liquid containing N,N'-methylenebisacrylamide (MBA) (1.5 wt.%) as the crosslinker and 2-hydroxy-4'-(2-hydroxyethoxy)-2-methylpropiophenone (Irgacure 2959) (1.5 wt.%) as the photoinitiator, AMH and APBA were dissolved at a molar ratio of 1.4 in the mixture. Similarly, the insulin (7 wt.%) formulation was prepared by dissolving AMH and APBA at a ratio of 2.6 in VP monomer liquid containing MBA (0.6 wt.%) and 2-hydroxy-4'-(2-hydroxyethoxy)-2-methylpropiophenone (1.5 wt.%). Then, a microneedle mask was molded by photopolymerization of VP and adjusted to cover three quarters of the patch 10 (the insulin loading) area. The glucagon formulation was deposited by pipette onto the unmasked part of the mold surface (silicon mold) and then placed under vacuum to infiltrate the needle-like recesses within the mold. The excess solution and mask were removed from the mold surface before photopolymerization under an ultraviolet lamp (100W; 365nm; Blak-Ray) for 5 min in an ice bath. Then, the insulin-loaded mixture was added to the remaining microneedle mold (previously covered by the mask), and excess solution was scraped with a blade followed by photopolymerized under the ultraviolet lamp for 8 min on ice. Afterward, the ultraviolet-curable material (Norland Optical Adhesive 86) for the base or substrate 12 was added dropwise onto the mold and spread evenly by covering with a 0.01-inch-thick, transparent polycarbonate film (McMaster-Carr). After cured under ultraviolet light for 15 min, the resulting therapeutic hybrid microneedle patch 10 was carefully separated from the mold and film, which was kept dry at room temperature for further study or use.

### Microneedle characterizations

The fluorescence image of the microneedles in the therapeutic hybrid microneedle patch 10 was tile scanned by confocal laser scanning microscopy (CLSM, LS880, ZESSI). ZEISS Supra 40VP field emission scanning electron microscope was used to characterize the therapeutic hybrid microneedle patch 10. The therapeutic hybrid microneedle patch 10 was sputtered with a gold/palladium target for 30 s before imaging. The mechanical strength of the microneedles 14a, 14b was assessed by an Instron 8516 tensile compression machine. The initial gauge was set to 2 mm between the microneedle tips 16 and the stainless-steel plate, with 10 N as the load cell capacity. The speed of the top stainless-steel plate movement towards the microneedles 14a, 14b was 0.25 mm/min. The failure force of the microneedles 14a, 14b was recorded when the needles began to buckle.

### In vitro release studies

To evaluate the glucose responsiveness of the insulin or glucagon formulation, the samples were incubated in 1 mL of PBS solution (pH 7.4) with various glucose concentrations (50, 100, 200, and 400 mg/dL) at 37 °C with gentle shaking (150 rpm). At predetermined time points, 30 µL of the supernatant was collected, and the released insulin or glucagon was quantified using a Coomassie (Bradford) protein assay (Thermo Fisher Scientific) in a 96 well plate. The absorbance was detected at 595 nm on the Infinite 200 Pro multimode plate reader (Tecan Group), and the concentration was calculated with insulin (15 µg/mL-1 mg/mL) or glucagon (8-500 µg/mL) standard curve. The co-release of the integrated insulin and glucagon formulation with the loading ratio of 3 to 1 was performed in the same way but measured with ELISA.

### Animal experiments

All animal experiments were performed in compliance with an animal study protocol approved by the Institutional Animal Care and Use Committee at University of California, Los Angeles. The *in vivo* performance of the patches was evaluated on streptozotocin-induced adult diabetic mice (male C57B6, age 8 wk; Jackson Laboratory). For microneedle insertion, the therapeutic hybrid microneedle patch 10 was pressed firmly for 10 s and immobilized on the mouse skin by applying a medical tape. To avoid movement, the mice were anesthetized with isoflurane during the application of the therapeutic hybrid microneedle patch 10. After insertion, the PGLs were recorded with an Accu-Chek Aviva (Roche Diabetes Care, Inc.) glucometer.

### Plasma insulin or glucagon level measurement

The plasma insulin or glucagon level was measured by collecting 20 µL of plasma, which was stored at -20°C until measurement using human insulin (catalog number KAQ1251)/glucagon (catalog number EHGCG) enzyme-linked immunosorbent assay (ELISA) kit (Thermo Fisher Scientific) following the manufacturer's instruction.

### Intraperitoneal glucose tolerance test (IPGGT)

For IPGTT triggered insulin release test, diabetic mice were treated with the insulin microneedle patch at a dose of 50 mg/kg. Intraperitoneal glucose (0.3 g/mL in PBS) was given at 4 hours after treatment at a dose of 3 g/kg to achieve an increased spike of blood glucose level, and PGLs were measured with an Accu-Chek Aviva blood glucose meter (Roche Diabetes Care, Inc.) through the tail vein blood (~3 µL). For plasma insulin quantification, blood was collected (~ 50 µL) at predetermined intervals, centrifuged to isolate plasma, and stored at -20°C until measurement with ELISA kit.

### Intraperitoneal insulin tolerance test (IPITT)

For IPITT-triggered glucagon release, insulin (70 U/kg) was given at two hours post glucagon microneedle patch insertion (17 mg/kg) to achieve a decrease of blood glucose. PGLs were measured with the glucose meter. Blood (~ 50 µL) was collected at selected time points to isolate plasma for glucagon analysis with the ELISA kit.

### H&E staining experiment

The glucagon and insulin therapeutic hybrid microneedle patches 10 were applied to the shaved backs of the mice for 10 h or 24 h. On days 1, 3, or 7 after microneedle removal, mice were euthanized, pieces of skin from the treated sites were harvested, and fixed in 4% formaldehyde for 24 hours before H&E staining by Translational Pathology Core Laboratory at Pathology & Laboratory Medicine, UCLA. Histopathology images were acquired on an Eclipse Ti2 fluorescence microscopy (Nikon).

### Statistical analysis

All of the results are presented as means ± S.D. Statistical analysis was performed using a two-tailed Student's t-test for two-group comparisons. All statistical analyses were performed using the Prism software package (Prism 7.0d; GraphPad Software, USA, 2017). The differences between experimental groups and control groups were considered statistically significant at *P* < 0.05. Significance is denoted in the figures as *P < 0.05, ***P* < 0.01, ****P <* 0.001, and ****P < 0.0001.

While embodiments of the present invention have been shown and described, various modifications may be made without departing from the scope of the present invention. The invention, therefore, should not be limited, except to the following claims, and their equivalents.

## Claims

1. A therapeutic hybrid microneedle patch (10) for delivering insulin and glucagon to living mammalian tissue comprising:
a base (12) having a plurality of microneedles (14a, 14b) extending away from the surface of the base (12), wherein a first plurality of microneedles (14a) comprise a biocompatible polymer loaded with insulin and a second plurality of microneedles (14b) comprise the biocompatible polymer loaded with glucagon.

2. The therapeutic hybrid microneedle patch (10) of claim 1, wherein the first plurality of microneedles (14a) and the second plurality of microneedles (14b) comprise sharpened tips.

3. The therapeutic hybrid microneedle patch (10) of claim 1, wherein the first plurality of microneedles (14a) comprise polymerized 3-(acrylamido)phenylboronic acid (APBA), 2-aminoethyl methacrylate hydrochloride (AMH), and 1-vinyl-2-pyrrolidinone (VP) containing insulin therein.

4. The therapeutic hybrid microneedle patch (10) of claim 1, wherein the second plurality of microneedles (14b) comprise polymerized 3-(acrylamido)phenylboronic acid (APBA), 2-aminoethyl methacrylate hydrochloride (AMH), and 1-vinyl-2-pyrrolidinone (VP) containing glucagon therein.

5. The therapeutic hybrid microneedle patch (10) of claim 1, wherein the number of the first plurality of microneedles (14a) comprise between 50% and 75% of the total number of first plurality of microneedles (14a) and second plurality of microneedles (14b).

6. The therapeutic hybrid microneedle patch (10) of claim 1, wherein the number of the first plurality of microneedles (14a) comprise about 75% of the total number of first plurality of microneedles (14a) and second plurality of microneedles (14b).

7. A therapeutic hybrid microneedle patch system for delivering insulin and glucagon to living mammalian tissue comprising:
a first patch (10a) comprising a base (12) having a plurality of microneedles (14a) extending away from the surface of the base (12), wherein the microneedles of the first patch (10a) comprise a biocompatible polymer loaded with insulin; and
a second patch (10b) comprising a base (12) having a plurality of microneedles (14b) extending away from the surface of the base (12), wherein the microneedles of the second patch (10b) comprise a biocompatible polymer loaded with glucagon.

8. The therapeutic hybrid microneedle patch system of claim 7, wherein the number of microneedles (14a) of the first patch (10a) is greater than the number of microneedles (14b) of the second patch (10b).

9. The therapeutic hybrid microneedle patch system of claim 7, further comprising one or more additional patches (10a, 10b) comprising a base (12) having a plurality of microneedles (14a, 14b) extending away from the surface of the base, wherein the microneedles of the one or more additional patches comprise a biocompatible polymer loaded with insulin or glucagon.

10. A method of manufacturing a therapeutic hybrid microneedle patch (10) for delivering insulin and glucagon to living tissue comprising:
providing a mold containing a plurality of needle-shaped cavities therein;
forming a mask over a portion of the mold;
applying a glucagon-containing solution containing 3-(acrylamido)phenylboronic acid (APBA), 2-aminoethyl methacrylate hydrochloride (AMH), 1-vinyl-2-pyrrolidinone (VP), glucagon, crosslinker, and photoinitiator over the non-masked portion of the mold;
removing the mask;
crosslinking the glucagon-containing solution with ultraviolet light;
applying an insulin-containing solution containing 3-(acrylamido)phenylboronic acid (APBA), 2-aminoethyl methacrylate hydrochloride (AMH), 1-vinyl-2-pyrrolidinone (VP), insulin, crosslinker, and photoinitiator over the portion of the mold that was previously masked;
crosslinking the insulin-containing solution with ultraviolet light;
applying a backing material over the mold and curing the backing material; and
separating the patch (10) from the mold.

11. The method of claim 10, wherein the crosslinker comprises *N,N'-*methylenebisacrylamide (MBA).

12. The method of claim 10, wherein the photoinitiator comprises 2-hydroxy-4'-(2-hydroxyethoxy)-2-methylpropiophenone (Irgacure 2959).

13. The method of claim 10, wherein the glucagon-containing solution and the insulating-containing solution are applied under vacuum.

## Patentansprüche

1. Ein therapeutisches hybrides Mikronadelpflaster (10) zur Abgabe von Insulin und Glukagon an lebendes Säugetiergewebe, umfassend:
eine Basis (12) mit einer Vielzahl von Mikronadeln (14a, 14b), die sich von der Oberfläche der Basis (12) weg erstrecken, wobei eine erste Vielzahl von Mikronadeln (14a) ein biokompatibles Polymer enthält, das mit Insulin beladen ist, und eine zweite Vielzahl von Mikronadeln (14b) das biokompatible Polymer enthält, das mit Glukagon beladen ist.

2. Das therapeutische hybride Mikronadelpflaster (10) nach Anspruch 1, wobei die erste Vielzahl von Mikronadeln (14a) und die zweite Vielzahl von Mikronadeln (14b) geschärfte Spitzen aufweisen.

3. Das therapeutische hybride Mikronadelpflaster (10) nach Anspruch 1, wobei die erste Vielzahl von Mikronadeln (14a) polymerisiertes 3-(Acrylamido)phenylboronsäure (APBA), 2-Aminoethylmethacrylat-Hydrochlorid (AMH) und 1-Vinyl-2-Pyrrolidon (VP) enthält, das Insulin darin enthält.

4. Das therapeutische hybride Mikronadelpflaster (10) nach Anspruch 1, wobei die zweite Vielzahl von Mikronadeln (14b) polymerisiertes 3-(Acrylamido)phenylboronsäure (APBA), 2-Aminoethylmethacrylat-Hydrochlorid (AMH) und 1-Vinyl-2-Pyrrolidon (VP) enthält, das Glukagon darin enthält.

5. Das therapeutische hybride Mikronadelpflaster (10) nach Anspruch 1, wobei die Anzahl der ersten Vielzahl von Mikronadeln (14a) zwischen 50% und 75% der Gesamtzahl der ersten Vielzahl von Mikronadeln (14a) und der zweiten Vielzahl von Mikronadeln (14b) umfasst.

6. Das therapeutische hybride Mikronadelpflaster (10) nach Anspruch 1, wobei die Anzahl der ersten Vielzahl von Mikronadeln (14a) etwa 75% der Gesamtzahl der ersten Vielzahl von Mikronadeln (14a) und der zweiten Vielzahl von Mikronadeln (14b) umfasst.

7. Ein therapeutisches hybrides Mikronadelpflastersystem zur Abgabe von Insulin und Glukagon an lebendes Säugetiergewebe, umfassend:
ein erstes Pflaster (10a) mit einer Basis (12) und einer Vielzahl von Mikronadeln (14a), die sich von der Oberfläche der Basis (12) weg erstrecken, wobei die Mikronadeln des ersten Pflasters (10a) ein biokompatibles Polymer enthalten, das mit Insulin beladen ist; und
ein zweites Pflaster (10b) mit einer Basis (12) und einer Vielzahl von Mikronadeln (14b), die sich von der Oberfläche der Basis (12) weg erstrecken, wobei die Mikronadeln des zweiten Pflasters (10b) ein biokompatibles Polymer enthalten, das mit Glukagon beladen ist.

8. Das therapeutische hybride Mikronadelpflastersystem nach Anspruch 7, wobei die Anzahl der Mikronadeln (14a) des ersten Pflasters (10a) größer ist als die Anzahl der Mikronadeln (14b) des zweiten Pflasters (10b).

9. Das therapeutische hybride Mikronadelpflastersystem nach Anspruch 7, weiter umfassend ein oder mehrere zusätzliche Pflaster (10a, 10b) mit einer Basis (12) und einer Vielzahl von Mikronadeln (14a, 14b), die sich von der Oberfläche der Basis weg erstrecken, wobei die Mikronadeln der ein oder mehrere zusätzlichen Pflaster ein biokompatibles Polymer enthalten, das mit Insulin oder Glukagon beladen ist.

10. Ein Verfahren zur Herstellung eines therapeutischen hybriden Mikronadelpflasters (10) zur Abgabe von Insulin und Glukagon an lebendes Gewebe, umfassend:
Bereitstellen einer Form, die eine Vielzahl von nadelförmigen Hohlräumen enthält;
Bilden einer Maske über einem Teil der Form;
Auftragen einer Glukagon-haltigen Lösung, die 3-(Acrylamido)phenylboronsäure (APBA), 2-Aminoethylmethacrylat-Hydrochlorid (AMH), 1-Vinyl-2-Pyrrolidon (VP), Glukagon, Vernetzer und Photoinitiator enthält, über den nicht maskierten Teil der Form;
Entfernen der Maske;
Vernetzen der Glukagon-haltigen Lösung mit ultraviolettem Licht;
Auftragen einer Insulin-haltigen Lösung, die 3-(Acrylamido)phenylboronsäure (APBA), 2-Aminoethylmethacrylat-Hydrochlorid (AMH), 1-Vinyl-2-Pyrrolidon (VP), Insulin, Vernetzer und Photoinitiator enthält, über den zuvor maskierten Teil der Form;
Vernetzen der Insulin-haltigen Lösung mit ultraviolettem Licht;
Auftragen eines Trägermaterials über die Form und Aushärten des Trägermaterials; und
Trennen des Pflasters (10) von der Form.

11. Das Verfahren nach Anspruch 10, wobei der Vernetzer N,N'-Methylenbisacrylamid (MBA) umfasst.

12. Das Verfahren nach Anspruch 10, wobei der Photoinitiator 2-Hydroxy-4'-(2-hydroxyethoxy)-2-methylpropiophenon (Irgacure 2959) umfasst.

13. Das Verfahren nach Anspruch 10, wobei die Glukagon-haltige Lösung und die Insulinhaltige Lösung unter Vakuum aufgetragen werden.

## Revendications

1. Un patch thérapeutique hybride à micro-aiguilles (10) pour administrer de l'insuline et du glucagon à un tissu mammalien vivant comprenant :
une base (12) ayant une pluralité de micro-aiguilles (14a, 14b) s'étendant à partir de la surface de la base (12), où une première pluralité de micro-aiguilles (14a) comprend un polymère biocompatible chargé en insuline et une seconde pluralité de micro-aiguilles (14b) comprend le polymère biocompatible chargé en glucagon.

2. Le patch thérapeutique hybride à micro-aiguilles (10) de la revendication 1, où la première pluralité de micro-aiguilles (14a) et la seconde pluralité de micro-aiguilles (14b) comprennent des pointes aiguisées.

3. Le patch thérapeutique hybride à micro-aiguilles (10) de la revendication 1, où la première pluralité de micro-aiguilles (14a) comprend de l'acide 3-(acrylamido)phénylboronique polymérisé (APBA), du chlorhydrate de méthacrylate de 2-aminoéthyle (AMH), et de la 1-vinyl-2-pyrrolidinone (VP) contenant de l'insuline.

4. Le patch thérapeutique hybride à micro-aiguilles (10) de la revendication 1, où la seconde pluralité de micro-aiguilles (14b) comprend de l'acide 3-(acrylamido)phénylboronique polymérisé (APBA), du chlorhydrate de méthacrylate de 2-aminoéthyle (AMH), et de la 1-vinyl-2-pyrrolidinone (VP) contenant du glucagon.

5. Le patch thérapeutique hybride à micro-aiguilles (10) de la revendication 1, où le nombre de la première pluralité de micro-aiguilles (14a) comprend entre 50% et 75% du nombre total de la première pluralité de micro-aiguilles (14a) et de la seconde pluralité de micro-aiguilles (14b).

6. Le patch thérapeutique hybride à micro-aiguilles (10) de la revendication 1, où le nombre de la première pluralité de micro-aiguilles (14a) comprend environ 75% du nombre total de la première pluralité de micro-aiguilles (14a) et de la seconde pluralité de micro-aiguilles (14b).

7. Un système de patch thérapeutique hybride à micro-aiguilles pour administrer de l'insuline et du glucagon à un tissu mammalien vivant comprenant :
un premier patch (10a) comprenant une base (12) ayant une pluralité de micro-aiguilles (14a) s'étendant à partir de la surface de la base (12), où les micro-aiguilles du premier patch (10a) comprennent un polymère biocompatible chargé en insuline ; et
un second patch (10b) comprenant une base (12) ayant une pluralité de micro-aiguilles (14b) s'étendant à partir de la surface de la base (12), où les micro-aiguilles du second patch (10b) comprennent un polymère biocompatible chargé en glucagon.

8. Le système de patch thérapeutique hybride à micro-aiguilles de la revendication 7, où le nombre de micro-aiguilles (14a) du premier patch (10a) est supérieur au nombre de micro-aiguilles (14b) du second patch (10b).

9. Le système de patch thérapeutique hybride à micro-aiguilles de la revendication 7, comprenant en outre un ou plusieurs patchs supplémentaires (10a, 10b) comprenant une base (12) ayant une pluralité de micro-aiguilles (14a, 14b) s'étendant à partir de la surface de la base, où les micro-aiguilles des patchs supplémentaires comprennent un polymère biocompatible chargé en insuline ou en glucagon.

10. Un procédé de fabrication d'un patch thérapeutique hybride à micro-aiguilles (10) pour administrer de l'insuline et du glucagon à un tissu vivant comprenant :
fournir un moule contenant une pluralité de cavités en forme d'aiguilles ;
former un masque sur une partie du moule ;
appliquer une solution contenant du glucagon, de l'acide 3-(acrylamido)phénylboronique (APBA), du chlorhydrate de méthacrylate de 2-aminoéthyle (AMH), de la 1-vinyl-2-pyrrolidinone (VP), du glucagon, un agent de réticulation et un photoinitiateur sur la partie non masquée du moule ;
retirer le masque ;
réticuler la solution contenant du glucagon avec de la lumière ultraviolette ;
appliquer une solution contenant de l'insuline, de l'acide 3-(acrylamido)phénylboronique (APBA), du chlorhydrate de méthacrylate de 2-aminoéthyle (AMH), de la 1-vinyl-2-pyrrolidinone (VP), de l'insuline, un agent de réticulation et un photoinitiateur sur la partie du moule qui était précédemment masquée ;
réticuler la solution contenant de l'insuline avec de la lumière ultraviolette ;
appliquer un matériau de support sur le moule et durcir le matériau de support ; et
séparer le patch (10) du moule.

11. Le procédé de la revendication 10, où l'agent de réticulation comprend du N,N'-méthylènebisacrylamide (MBA).

12. Le procédé de la revendication 10, où le photoinitiateur comprend du 2-hydroxy-4'-(2-hydroxyéthoxy)-2-méthylpropiophénone (Irgacure 2959).

13. Le procédé de la revendication 10, où la solution contenant du glucagon et la solution contenant de l'insuline sont appliquées sous vide.
